(19) Europäisches Patentamt
European Patent Office
Office européen des brevets

(11) **EP 1 893 266 B1**

(12) **EUROPEAN PATENT SPECIFICATION**

(45) Date of publication and mention
of the grant of the patent:
**18.05.2016 Bulletin 2016/20**

(21) Application number: **06747935.2**

(22) Date of filing: **20.06.2006**

(51) Int Cl.:
*A61M 16/00* *(2006.01)*        *A61B 5/087* *(2006.01)*

(86) International application number:
**PCT/SE2006/000743**

(87) International publication number:
**WO 2006/137784 (28.12.2006 Gazette 2006/52)**

(54) **APPARATUS, METHOD AND COMPUTER PROGRAM FOR LEAKAGE COMPENSATION FOR A VENTILATOR**

GERÄT, VERFAHREN UND COMPUTERPROGRAMM ZUR LECK-KOMPENSIERUNG FÜR EIN VENTILATOR

APPAREIL, PROCÉDÉ ET PROGRAMME INFORMATIQUE DESTINÉS À UNE COMPENSATION DE FUITE POUR UN VENTILATEUR

(84) Designated Contracting States:
**AT BE BG CH CY CZ DE DK EE ES FI FR GB GR HU IE IS IT LI LT LU LV MC NL PL PT RO SE SI SK TR**

(30) Priority: **21.06.2005   SE 0501500**

(43) Date of publication of application:
**05.03.2008   Bulletin 2008/10**

(73) Proprietor: **Breas Medical AB**
**435 33 Mölnlycke (SE)**

(72) Inventor: **TIEDJE, Mikael**
**425 30 Hisings Backa (SE)**

(74) Representative: **Valea AB**
**Box 1098**
**405 23 Göteborg (SE)**

(56) References cited:
EP-A2- 0 722 747        WO-A1-02/28460
WO-A1-98/06449        US-A- 5 660 171
US-A1- 2002 014 240        US-A1- 2002 023 645
US-A1- 2005 020 932        US-B1- 6 305 374

## Description

### *Field of invention.*

The present invention relates to a method for determining the current leakage present in a ventilator and method for compensating for this leakage.

### *Background of the invention*

Patients suffering from different forms of breathing disorders can be subject to several types of treatments depending on the illness or disorder present. Such treatments include surgical procedures, pharmacologic therapy, and non-invasive mechanical techniques. Surgical techniques to remedy breathing disorders constitute a considerable risk for the patient and can lead to permanent injury or even mortality. Pharmacologic therapy has in general proved disappointing with respect to treating certain breathing disorders, e.g. sleep apnea. It is therefore of interest to find other treatments, preferably non-invasive techniques.

A mechanical ventilator represents a non-invasive technique for treatment of certain breathing disorders such as ventilatory failure, hypoventilation, and periodic breathing during sleep and awake and in sleep apnea that occurs exclusively during sleep. Ventilatory failure includes all forms of insufficient ventilation with respect to metabolic need whether occurring during wake or periods of sleep. Hypoventilation and periodic breathing, in its most frequently occurring form referred to as Cheyne-Stokes ventilation, may occur periodically or constantly during wake or sleep. Conditions associated with hypoventilation, in particular nocturnal hypoventilation include e.g. central nervous system disorders such as stroke, muscular dystrophies, certain congenital conditions, advanced chronic obstructive pulmonary disease (COPD), etc. Cheyne-Stokes ventilation or various forms of central apnea are commonly associated with cardiac and circulatory disorders, in particular cardiac failure.

Ventilatory failure is a potentially life threatening condition. The general comorbidity in patients with failing ventilation is considerable. The condition is highly disabling in terms of reduced physical capacity, cognitive dysfunction in severe cases and poor quality of life. Patients with ventilatory failure therefore experience significant daytime symptoms but in addition, the majority of these cases experience a general worsening of their condition during state changes such as sleep. The phenomenon of disordered breathing during sleep, whether occurring as a consequence of ventilatory failure or as a component of sleep apnea in accordance with the description above causes sleep fragmentation. Daytime complications include sleepiness and cognitive dysfunction. Severe sleep disordered breathing occurring in other comorbid conditions like obesity, neuromuscular disease, post polio myelitis states, scoliosis or heart failure may be associated with considerable worsening of hypoventilation and compromised blood gas balance. Sleep apnea has been associated with cardiovascular complications including coronary heart disease, myocardial infarction, stroke, arterial hypertension, thrombosis, and cardiac arrhythmia. It is therefore of both immediate and long-term interest to reduce the exposure to sleep disordered breathing.

Recent advancement in mechanical non-invasive ventilator techniques includes administration of continuous positive airway pressure (CPAP) in different forms of sleep disordered breathing. During CPAP administration an elevated airway pressure is maintained throughout the breathing phase during a period coinciding with sleep. In sleep apnea this procedure may provide appropriate stabilization of the upper airway thereby preventing collapse. This, so called mono-level CPAP therapy, provides an almost identical pressure during inhalation and exhalation. Not only may CPAP be uncomfortable for the patient due to a sensed increased work of breathing during ventilation, specifically expiration. Some forms of apnea, mainly including those of central origin, and most forms of hypoventilation are only poorly controlled by CPAP. A more recently developed bi-level CPAP system administers different pressure levels during inhalation and exhalation. Bi-level CPAP provides increased comfort for most patients and not infrequently, an improved clinical response. Bi-level CPAP provides two pressure levels, Inspiratory Positive Airway Pressure (IPAP) and Expiratory Positive Airway Pressure (EPAP). IPAP is administered during the inhalation phase while EPAP is given during the exhalation phase.

All ventilator systems exhibit leakage during administration of pressurized breathing gas and a suitable method for measuring the current leakage and compensating for the same is of interest. Several systems exists that measures and compensates for the leakage present in the ventilato/human setup.

Some methods are using some specific sample points as references and thus depend strongly on the sample interval of the detection system. With a limited sample frequency there is a risk that the exact breathing cycle point is missed and a measurement is done slightly away from the correct point and thus a measurement is made that contains an error. Other systems determine the shift of the overall breathing cycle from a baseline. These systems typically give unstable feedback giving a compensation that moves up and down slowly continuously.

One such method is described in the US patent nr 6 945 248. where a method and apparatus for determining leak and respiratory airflow are disclosed. The non-linear conductance of a leak path is estimated dividing a low pass filtered instantaneous airflow by the low pass filtered square root of the instantaneous pressure. The value of the instantaneous leak is then obtained by multiplying the non-linear conductance by the square root of the instantaneous pressure. Finally, the respiratory air flow is calculated as the difference between the instantaneous air flow and the instantaneous leak flow, However, since an instantaneous leak flow is calculated from measured instantaneous values for the air-flow and the pressure, this method will suffer from the aforementioned deficiencies connected to unstable feedback.

Another example of a current system is provided in international Patent Application WO 02/26460 which is directed towards a medical ventilator system and method that riggers, cycles, or both based on patient effort, which is determined from cross-correlating patient flow and patient pressure. The medical ventilator is also controlled such that sensitivity to a patient initiated trigger Increases as the expiratory phase of the breathing cycle progresses. This example system also provides adaptive adjustment of cycling criteria to optimize the cycling operation.

The object of the invention is to overcome some of the deficiencies associated with known technology.

### *Summary of the Invention*

This object is achieved by a ventilator for supplying pressurized breathing gas which comprises a flow generator for producing pressurized breathing gas to be delivered to an interface, a device for delivering the pressurized breathing gas to a patient, a first interface connected to the flow generator and arranged to deliver the breathing gas to a patient, at least one second interface connected to a processing unit and adapted to receive at least one signal indicative of the flow of pressurized breathing gas from the patient and to deliver the signal to a processing unit and a processing unit for controlling the pressure from the ventilator based on the signal received from the second interface, where the processing unit is arranged to compensate for leakage in the ventilator by using a ratio between the measured flow of pressurized breathing gas and a flow related to a reference standard leak.

In one embodiment of the invention the at least one first interface connected to the flow generator and arranged to deliver the breathing gas to a patient may be located in said ventilator.

in one embodiment of the invention, the first Interface for delivering the pressurized breathing gas to a patient may be connected to tubing or any other type of closed gas conductor suitable for delivering the pressurized breathing gas to the patient.

In another embodiment of the invention the at least one second interface connected to a processing unit and adapted to receive at least one signal indicative of the flow of pressurized breathing gas from the patient and to deliver the signal to a processing unit.

As an option, the second interface above may also be arranged to receive signals indicative of the physiological state of the patient which for example may be data obtained from EEG, EMG, EOG and ECG-measurements, data indicative of the patient's eye movements, body temperature and other data suitable for characterizing the physiological state of the patient.

The first and second interfaces may for example be wired or wireless interfaces.

Also, processing unit may additionally comprise a computational device for analyzing the data received from the second interface. This computational device may also calculate the standard reference leak flow mentioned above by using the Bernoulli's equation for a stream in a tube and the fact that the energy going Into the tube is equal to the energy going out of the tube. The mass flow may then be calculated according to the following formula:

$$m = \int_{A_c} \rho u(r, x) dA_c$$

where m is the mass flow through a pipe, $\rho$ the volume density of the fluid in the pipe, $u(r,x)$ the velocity profile for the fluid in the pipe and $A_c$ the cross sectional area for the flow) and where said calculated mass flow is divided by pressure for said pressurized breathing gas to obtain a normalized mass flow.

Of course the computational device may also be adapted to retrieve values for the standard reference leak flow from a table of values representing the standard reference leak flow at a certain pressure for the pressurized breathing gas.

This approach would have the advantage of accelerating the calculation of the ratio between the measured instantaneous mass flow and the standard reference leak flow.

In a further embodiment of the ventilator according to the present invention, the processing unit may additionally comprise a data storage unit for later analysis and inspection of the measured signals delivered by the second interface indicative of the instantaneous mass flow for the pressurized breathing gas, the physiological state of the patient and the aforementioned ratio between the measured signal indicative of the instantaneous mass flow for the pressurized breathing gas and a standard reference leak flow. This date storage unit may be a non-volatile memory device, such as for example a hard-disk or some other type of suitable memory device.

In yet another embodiment of the invention the processing unit above may include a first communication device for communication with an external sensing device, such as a flow sensor. Also, the processing unit above may additionally include a second communication device for communication with the ventilator from en external computational device for retrieving data and results for analysis and/or inspection.

These communication devices may be wired or wireless communication devices and may work according to different connection standards for wired or wireless communication.

In another aspect of the present invention a ventilation system is provided, which comprises a mechanical ventilator for supplying pressurized breathing gas, a tubing for guiding the pressurized breathing gas connected to the mechanical ventilator, a device connected to the tubing for administrating the pressurized breathing gas to a patient,

at least one sensing device arranged to measure at least a signal indicative of the instantaneous flow for the pressurized breathing gas and further arranged to send the signal to the mechanical ventilator and a processing unit arranged to receive the signal indicative of flow for controlling the pressure or flow from the mechanical ventilator, where the processing unit Is arranged to compensate for leakage In the ventilator system using a ratio between said measured flow of pressurized breathing gas and a flow related to a reference standard leak.

In one embodiment of the invention, the sensing device above may comprise a flow sensor.

This flow sensor may be located either in or nearby the mechanical ventilator mentioned above or nearby the device connected to the tubing for administrating the pressurized breathing gas to a patient mentioned above.

One may also arrange two such flow sensors, one nearby the second interface for receiving at least one signal Indicative of the flow of pressurized breathing gas. In this fashion one could measure the flow of the breathing gas by calculating the difference between the flow measured by the flow sensor near the mechanical ventilator and the flow measured by the sensor near the device for delivering the pressurized gas to the patient, which for example may be a face mask or the like.

In another embodiment of the invention the device connected to the tubing for administrating the pressurized breathing gas to a patient may be a breathing mask, where such a breathing mask may cover the face or the nose of the patient. Also, the mask may only cover the nose or the nostrils of the patient. However, instead of using such a mask, it is also possible to use a hood covering a part or the whole of the patient's body.

The advantage of a mask would be the relatively easy positioning of the mask on the patients face and the small cost involved in using face masks.

One advantage of using the hood would be an even better control of the leakage occurring due to the imperfect fit of the mask or hood administering pressurized breathing gas to the patient.

In yet another aspect of the present invention a method for determining current leakage in a ventilator is provided, where the method comprises the steps of

- measuring the mass flow through the ventilator
- comparing values from a standard leak calculation for a standard leak in the ventilator, where a ratio between the measured mass flow through the ventilator and the values from a standard leak calculation for a standard leak flow in the ventilator is calculated and where the difference between the measured mass flow and the calculated standard leak flow is compensated for and the current leakage from said comparison is determined.

It is also contemplated to use the calculated ratio between the measured mass flow through the ventilator and the values from a standard leak calculation for a standard leak in the ventilator as basis for compensation for the difference between the measured mass flow and the calculated standard leak flow.

In one embodiment of the method according to the present invention some further substeps may be included, such as the sampling instantaneous values for the mass flow through the ventilator and calculation of a ratio between each sampled value for the instantaneous mass flow and a corresponding value for the standard leak flow.

Further step may also provide for sampling values for the mass flow through the ventilator during one predetermined time period, calculating a ratio between sampled mass flow values above and corresponding standard leak flow values during said predetermined time period, calculating a mean value for the ratio by integrating the ratio over the predetermined time period measured and dividing it by the number of flow values sampled and calculating mass flow through the ventilator using a known relation between the mean value for the flow ratio and a standard leak flow.

The standard leak flow may thereby be calculated from Bemoulli's equation along a stream in a tube and the use of the energy conservation principle as already explained previously.

The efficiency of the method described above may be further enhanced by calculating the mean value for the aforementioned ratio according to the steps of:

- calculating a volume for the inspiration- and the expiration phases of a patient
- determining the volume difference between the inspiration- and expiration phases of the patient
- calculating the actual flow rate based on the volume difference
- calculating a ratio between the actual flow rate based on the volume difference and a standard leak flow and adding the ratio between the actual flow rate based on said volume difference and a standard leak flow and the mean value for said ratio obtained through integration over a predetermined time period as described previously. Thus, the value for the volume difference between the inspiration- and expiration phases of the patient can be used to further enhance the stability of the feedback to compensate for leakage and to hold the compensation stable if the leakage is changed during operation.

The method according to the present invention is especially suited to be implemented by the ventilator and the ventilation system described above.

In yet another aspect of the present invention a computer program for determining a leakage in a ventilator system is provided, where the computer program comprises instruction sets for obtaining data indicative of a first mass flow of breathing gas through the ventilator system, obtaining the first mass flow through the ventilator and the second standard leak flow in the ventilator system and an instruction set for determining a leakage in the ventilator system.

The computer program is specially suited to implement the method steps indicated above and to receive signals from and to control building parts included in the ventilator and the ventilation system according to the invention.

### Brief description of figures

In the following the invention will be described in a non-limiting way and in more detail with reference to exemplary embodiments illustrated in the enclosed drawings, in which:

> Fig. 1 illustrates schematic of a breathing circuit system according to the present invention;
>
> Fig. 2 is a schematic block diagram of a ventilator apparatus according to the present invention;
>
> Fig. 3 illustrates a measured and standard flow curve versus pressure;
>
> Fig. 4 illustrates a flow schematic according to the present invention;
>
> Fig. 5 illustrates a schematic breathing cycle;
>
> Fig. 6 illustrates a schematic block diagram of a method according to the present invention; and
>
> Fig. 7 illustrates in a schematic block diagram another embodiment of the method according to the present invention.

### Detailed description

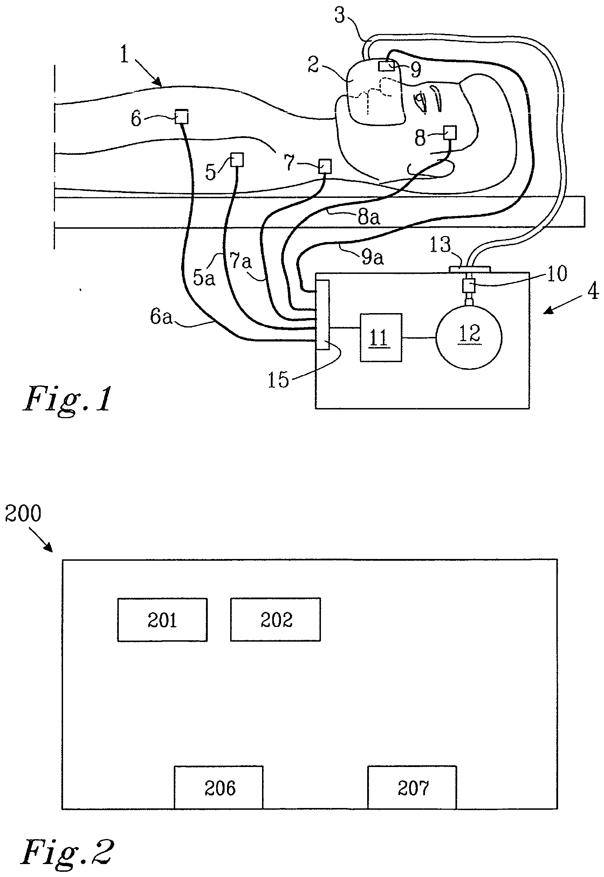

In Fig. 1 a schematic mechanical ventilation system used for the treatment of hypoventilation disorders is depicted. A ventilation system comprise a mechanical ventilator 4 supplying pressurized breathing gas, tubing 3 for guiding breathing gas to the patient 1, a breathing mask 2 or similar for administrating the breathing gas to the patient 1. sensing means 5, 6, 7, 8, 9 and 10 for determining the physiological status of the patient 1. The number of sensors connected to the mechanical ventilator may be one or more; however, in a preferred embodiment of the present invention at least one sensor is necessary: a breathing gas flow measurement which may be located essentially anywhere along the breathing gas tubing or in the mask. A mechanical ventilator 4 is supplying breathing gas for instance as a positive airway pressure via a tubing 3 and through a mask 2 to a patient 1. The mask 2 can be a face mask 2 covering both the mouth and nose or a nasal mask covering only the nose or nostrils depending on the patients needs. It can also be a hood covering the complete head or body of the patient.

The breathing gas may be of any suitable gas composition for breathing purposes as understood by the person skilled in the art, the composition may depend on the physiological status of the patient and the treatment of interest

The pressure or flow from the ventilator 4 Is controlled by a processing unit 11 as shown In Fig. 1. The processing unit 11 may involve a computer program that receives one or several input parameters 5, 6, 7, 8, 9, and 10 obtained from the patient 1 describing the physiological status of the patient and pressure/flow data indicative of breathing gas system configuration and status. Data indicative of patient status is obtained using sensors 5. 6, 7, 8, 9, and 10 connected to the patient and transferred to the processing unit 11 via connection means 5a, 6a, 7a, 8a, and 9a (connection means for sensor 10 is not depicted in Fig. 1 since the sensor may be placed at several different locations, such as inside the ventilator apparatus) and a second interface (15) in the ventilator (4). These input parameters may be for instance flow or pressure signals, data obtained from EEG, EMG, EOG. and ECG measurements. $O_2$ and/or $CO_2$ measurements In relation to the patient, body temperature, blood pressure, SpO2 (oxygen saturation), eye movements, and sound measurements. It should be understood that the invention is not limited to the above mentioned input parameters but other input parameters may be used. In Fig. 1 not all sensors 5, 6, 7, 8, 9, and 10 and sensor connection means 5a, 6a, 7a, 8a, and 9a are depicted, only a subset is shown in order to illustrate a schematically view of the system and the depicted locations are only given as examples and are In no way limiting to the invention, e.g. the flow signal may be measured at either the mask location or close to the mechanical ventilator or at both locations in order to deduce a differential signal if this is required.

The flow sensor 10 may be located at several different positions, e.g. in the breathing air tubing 3 at any suitable position, such as close to the mechanical ventilator apparatus (or even within the ventilator housing) or in the vicinity of the mask.

The input data is then supplied to a processing unit 11 via the second interface (15).

In figure 2, the processing unit 200 comprises at least computational means 201, where the computational or processing means 201 analyses the measured data, preferably data from the flow measurement, according to an appropriate method, algorithm or algorithms (to be discussed in detail below) in order to determine an appropriate response and send control signal or signals to a mechanical ventilator unit 12. This mechanical ventilator unit 12 may be a fan 12 arranged to deliver appropriate amounts of breathing gas at specified and controlled pressure levels. The processing means may for instance be a microprocessor, computer, workstation. FPGA (Field programmable array), or ASIC (Application Specific Integrated Circuit). The processing unit may be built into the ventilator or be located external of the ventilator in a stand alone unit.

The processing unit 200 may also comprise a data storage unit 202 for post analysis and inspection and also a connection for an internal or external non-volatile memory device, like for instance a memory device using a USB connection, an external hard drive, a floppy disk, a CD-ROM writer, a DVD writer, a Memory stick, a Compact Flash memory, a Secure Digital memory, an xD-Picture memory card, or a Smart Media memory card. These are only given as examples, and are not limiting for the Invention, many more non-volatile memory devices may be used In the invention as appreciated by the person skilled in the art.

The mechanical ventilator 12 may also have input means (not shown) for manually setting control parameters and other parameters necessary for the operation of the device.

Through a first and a second communication means 206 and 207 illustrated in figure 2 it is possible to communicate with the device 4 to and from an external computational device or one of the flow sensors (5, 6, 7, 8, 9, 10) for retrieving data and results for immediate and/or later analysis and/or inspection. The communication means can be of a serial type like for instance according to the standards RS232, RS485, USB. Ethernet, or Fire wire, or of a parallel type like for instance according to the standards Centronics, ISA, PCI, or GPIB/HPIB (General purpose interface bus). It may also be any wireless system of the standards in the IEEE 802.11, 802.15, and 802.16 series, HiperLAN, Bluetooth, IR, GSM, GPRS, or UMTS, or any other appropriate fixed or wireless communication system capable of transmitting measurement data. It can also be of any proprietary non-standardized communication formats, whether it is wireless or wired.

The ventilator device 4 may also have display means (not shown) for displaying measured data and obtained response parameters for use by a physician, other medical personnel, or the patient. The display means may be of any normal type as appreciated by a person skilled in the art. The data is displayed with such a high rate that a real time feedback is provided to a person monitoring the ventilator characteristics and function for immediate feedback and control.

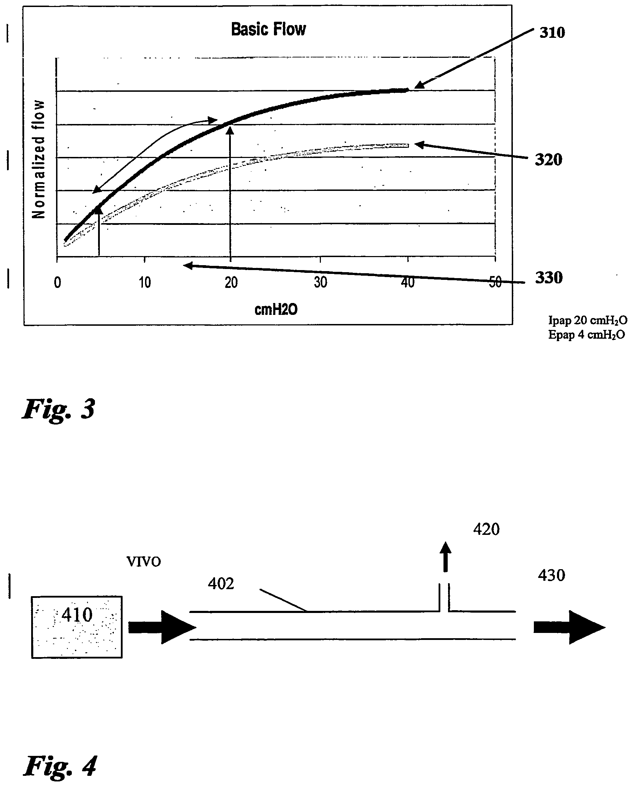

Fig. 4 is a schematic of flow related issues in a ventilator/human setup, i.e. a ventilator connected to a patient. A ventilator is connected to a hose or tubing 402 delivering a pressurized breathing gas; this hose 402 is In turn connected to a patient 430 using a suitable mask or similar device. However, a leak 420 may be present, for instance due to that the mask does not fit exactly to the patient 430 or the patient 430 has the mouth opened slightly.

The current flow is sampled at the ventilator side of the hose or within the ventilator with a certain frequency and In each sample point a ratio between the measured flow and a reference standard leak flow is determined (however, the flow may also be optionally measured at the mask side of the ventilator system). This difference between the measured flow and the standard leak flow is shown in Fig. 3, where the upper curve shows the measured flow 310 and the lower curve the calculated flow for a standard leak 320 at a certain pressure. The area bordered by the curved and the two straight arrows depicts the measured flow 310 for one breathing cycle 330.

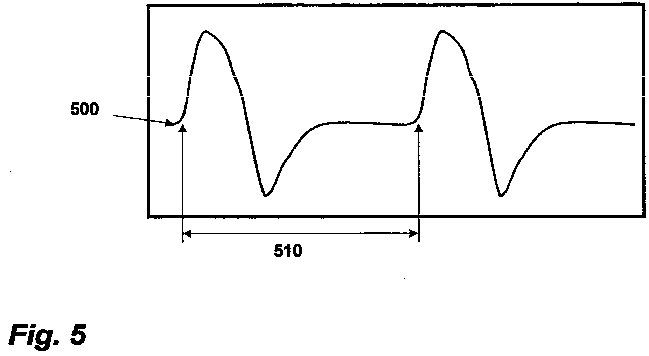

This series of ratio measurements is shown in Fig. 5 for two breathing cycles. 510 depict the start of the inspection of the ratio measurements and 510 the average calculation period, which in this case ts the length of breathing cycle of the patient. An average of a breathing cycle can than be determined by integrating over a cycle and dividing with the integration number (i.e. number of samples). By adding or subtracting the mean value from the flow control parameter it Is possible to compensate for this average error determined from the ratio calculation. This can be done by adding the necessary flow to the entire breathing cycle.

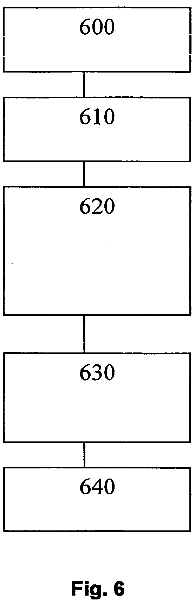

In an embodiment of the present invention, a method is provided for determining the flow leak and compensating for the same as shown in Fig. 6, this method can be implemented both in hardware and in software as understood by the person skilled In the art. At step 600 the sampling of data is started and sample points from the breathing cycle of the patient are gathered.

At the next step 610 a ratio between the measured instantaneous mass flow for the pressurized air delivered to the patient and the calculated reference leak flow at a certain pressure is built. The values for the reference leak flow at a certain pressure may be stored in a table and simply accessed when calculating the ratio above.

In case one is interested in measuring the ratio over a full breathing cycle of the patient, a mean ratio is calculated at step 620, where the ratio is integrated over a full breathing cycle of the patient and divided by the number of samples taken during the breathing cycle.

The mass flow for the pressurized air is then calculated at step 630, where a known relation for the ratio between the measured instantaneous mass flow for the pressurized air and the reference leak flow and the reference leak flow is used.

If the mass flow for the pressurized breathing gas has changed since the last measurement, the trigger baseline for the breathing cycle of the patient is adjusted at step 640, either upwards or downwards depending on whether the mass flow has decreased or increased.

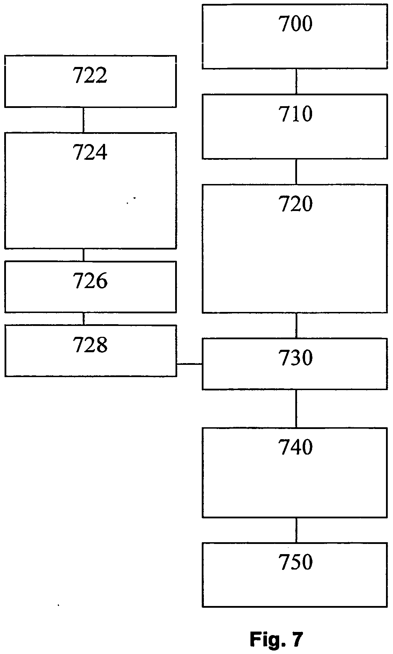

In another embodiment of the method according to the present invention shown in Fig. 7, the above mentioned method is combined with a volume measurement method. It should be mentioned that steps 700 to 720 are identical with the steps 600 to 620 from figure 6.

At step 722, the total volume of the pressurized gas administered to the patient is calculated. Then, at step 724 the difference between the volume of the pressurized breathing gas during the inspiration and the expiration phases of the patient is calculated, which is used at step 726 to calculate the flow rate of the pressurized breathing gas.

At step 728, a ratio delta is calculated between the flow rate during the inspiration and the expiration phases of the patient.

Finally, at step 730, the ratio delta above is added to the mean ratio between the measured instantaneous mass flow and the standard reference leak flow for the pressurized breathing gas.

The use of the extra delta parameter servers to further enhance the stability of the feedback to compensate for leakage and hold the compensation stable if the leakage is changed during operation. The system will determine the leakage and adjust the control parameters in such a way that it will be compensated for in a few breathing cycles.

It should be noted that the word "comprising" does not exclude the presence of other elements or steps than those listed and the words "a" or "an" preceding an element do not exclude the presence of a plurality of such elements. It should further be noted that any reference signs do not limit the scope of the claims, that the invention may at least in part be implemented by means of both hardware and software, and that several "means" may be represented by the same item of hardware.

The above mentioned and described embodiments are only given as examples and should not be limiting to the present invention. Other solutions, uses, objectives, and functions within the scope of the invention as claimed in the below described patent claims should be apparent for the person skilled in the art.

**Claims**

1. A ventilator (4) for supplying pressurized breathing gas, comprising:

   - a flow generator (12) for producing pressurized breathing gas to be delivered to a patient;
   - a first interface (13) connected to said flow generator (12) and arranged to receive said pressurized breathing gas from said flow generator (12) and to deliver said pressurized breathing gas to a patient (1);
   - at least one second interface (15) connected to a processing unit (11, 200) and adapted to receive at least one signal indicative of a measured flow of pressurized breathing gas from the patient (1) and to deliver the signal to said processing unit (11. 200), and: said processing unit (11, 200) for controlling the pressure from the ventilator (4) based on the signal indicative of the flow of said pressurized breathing gas received from said second interface (15), said processing unit (11, 200) being arranged to compensate for leakage in said ventilator (4)

**characterized in that** to compensate for leakage in said ventilator a ratio is used between said measured flow of pressurized breathing gas and a flow related to a reference standard leak.

2. Ventilator according to claim 1, wherein said at least one first interface (13) for delivering said pressurized breathing gas to a patient is located in said ventilator (4).

3. Ventilator according to claims 1 or 2, wherein said processing unit (11, 200) further comprises a computational device (201) adapted to calculate mass flow for a standard leak using a formula derived from Bemoulli's equation, said formula being:

$$m = \int_{A_e} \rho u(r, x) dAc$$

where $m$ is the mass flow through a pipe, $\rho$ the volume density of the fluid in the pipe, $u(r,x)$ the velocity profile for the fluid In the pipe and $A_c$ the cross sectional area for the flow, and where said calculated mass flow is divided by pressure for said pressurized breathing gas to obtain a normalized mass flow.

4. Ventilator according to claim 3, wherein said computational device (201) is adapted to retrieve values for said standard reference leak flow from a table of values representing said standard reference leak flow values at a certain pressure for the pressurized breathing gas.

5. Ventilator according to one of the claims 1 or 4, wherein said processing means (11. 200) additionally comprises a data storage unit (202) for later analysis and inspection of the measured signals indicative of the instantaneous mass flow for the pressurized breathing gas, the physiological state of the patient (1) and said ratio between the measured signal indicative of the instantaneous mass flow for the pressurized breathing gas and a standard reference leak flow.

6. Ventilator according to dalm 1, wherein said processing unit (11, 200) additionally comprises a first communication device (206) for communicating with an external sensing device (5, 6, 7, 8, 9, 10).

7. Ventilator according to claim 1, wherein said processing unit (11, 200) further comprises a second communication device (207) for communication with the ventilator (4) from an external computational device for retrieving data and results for analysis and/or inspection.

8. Ventilator according to claims 6 or 7, wherein said first or second communication devices (206, 207) may be a wired or a wireless communication device (207).

9. Ventilation system comprising

    - the mechanical ventilator (4) of claim 1.
    - a tubing (3) for guiding said pressurized breathing gas connected to said mechanical ventilator (4),
    - a device (2) connected to said tubing (3) for administrating said pressurized breathing gas to a patient (1).
    - at least one sensing device (5, 6, 7, 8, 9, 10) arranged to measure at least a signal indicative of the instantaneous flow for said pressurized breathing gas and further arranged to send said signal to said mechanical ventilator (4).

10. Ventilation system according to claim 9, **characterized by** that said at least one sensing device (5, 6, 7, 8, 9, 10) for measuring a signal indicative of the instantaneous flow for said pressurized breathing gas is located in or nearby said mechanical ventilator (4) or nearby said device (2) for administering said pressurized breathing gas to a patient (1).

11. Ventilation system according to any of claims 9 to 10, wherein said processing unit (11, 200) further comprises a computational device (201) adapted to calculate said mass flow for a standard leak using a formula derived from Bernoulli's equation, said formula being:

$$m = \int_{A_c} \rho u(r,x) dAc$$

where m is the mass flow through a pipe. $\rho$ the volume density of the fluid in the pipe, $u(r,x)$ the velocity profile for the fluid in the pipe and $A_c$ the cross sectional area for the flow, and where said calculated mass flow is divided by pressure for said pressurized breathing gas to obtain a normalized mass flow.

12. Method for determining a leakage in a ventilator, comprising the steps of:

    - measuring the mass flow through the ventilator;
    - calculating a reference standard leak flow in said ventilator;

**characterized by**
calculating a ratio between said measured mass low through the ventilator and said calculated reference standard leak flow in said ventilator; and determining said leakage from said ratio.

13. Method according to claim 12, wherein based on said calculated ratio between the measured mass flow through the ventilator and said values from a standard leak calculation for a standard leak in said ventilator, a compensation for the difference between the measured mass flow and the calculated standard leak flow is performed.

14. Method according to claim 12, wherein said step of measuring the mass flow through the ventilator further comprises the sub steps of:

    - sampling instantaneous values for the mass flow through the ventilator, and
    - calculating a ratio between said each sampled value for the instantaneous mass flow and a corresponding value for the standard leak flow.

15. Method according to claim 12 or 14, wherein said sub steps of sampling said instantaneous values for the mass flow through the ventilator and calculation of said ratio further comprises the steps of:

    - sampling values for the mass flow through the ventilator during one predetermined time period;
    - calculating a ratio between said sampled mass flow values and corresponding standard leak flow values during said predetermined time period;
    - calculating a mean value for said ratio by integrating the ratio over the predetermined time period measured and dividing it by the number of flow values sampled; and
    - calculating mass flow through the ventilator using a known relation between said mean value for the flow ratio and a standard leak flow.

16. Method according to claim 12, wherein said calculation for the standard leak flow in said ventilator is performed from Bernoulli's equation.

17. Method according to claim 12, wherein said mass flow for a standard leak Is calculated using a formula derived from Bernoulli's equation, said formula being:

$$m = \int_{A_c} \rho u(r,x) dAc$$

where $m$ is the mass flow through a pipe, $\rho$ the volume density of the fluid in the pipe, $u(r,x)$ the velocity profile for the fluid In the pipe and $A_c$ the cross sectional area for the flow, and where said calculated mass flow is divided by pressure for said pressurized breathing gas to obtain a normalized mass flow.

18. Method according to claim 12, wherein said step of calculating the mean value for said ratio further includes the sub steps of:

- calculating a volume for the inspiration- and the expiration phases of a patient;
- determining a volume difference between said inspiration- and expiration phases:
- calculating the actual flow rate based on said volume difference:
- calculating a ratio between said actual flow rate based on said volume difference and a standard leak flow; and
- adding said ratio between the actual flow rate based on said volume difference and a standard leak flow and said mean value for said ratio.

19. A computer program adapted to perform the method of claim 12, comprising instruction sets for:

- obtaining data indicative of a first mass flow of breathing gas through the ventilator system:
- obtaining a second mass flow for a standard leak flow in said ventilator system;
**characterized by**
- calculating a ratio between said first mass flow and said second standards leak flow in said ventilator system and;
- determining a leakage in said ventilator system from said ratio.

**Patentansprüche**

1. Ventilator (4), um unter Druck stehendes Atemgas zuzuführen, der umfasst:

- einen Strömungsgenerator (12), um unter Druck stehendes Atemgas, das an einen Patienten abgegeben werden soll, herzustellen;
- eine erste Schnittstelle (13), die mit dem Strömungsgenerator (12) verbunden ist und angeordnet ist, um das unter Druck stehende Atemgas aus dem Strömungsgenerator (12) aufzunehmen und um das unter Druck stehende Atemgas an einen Patienten (1) abzugeben;
- mindestens eine zweite Schnittstelle (15), die mit einer Verarbeitungseinheit (11, 200) verbunden ist und dafür ausgelegt ist, um mindestens ein Signal zu empfangen, das einen Hinweis auf eine gemessene Strömung eines unter Druck stehenden Atemgases von dem Patienten (1) gibt, und um das Signal an die Verarbeitungseinheit (11, 200) abzugeben, und;

die Verarbeitungseinheit (11, 200), um den Druck von dem Ventilator (4) auf der Grundlage des Signals, das einen Hinweis auf die Strömung des unter Druck stehenden Atemgases gibt, das von der zweiten Schnittstelle (15) empfangen wird, zu steuern, wobei die Verarbeitungseinheit (11, 200) angeordnet ist, um ein Leck in dem Ventilator (4) zu kompensieren,
**dadurch gekennzeichnet,**
**dass**, um das Leck in dem Ventilator zu kompensieren, ein Verhältnis zwischen der gemessenen Strömung des unter Druck stehenden Atemgases und einer Strömung verwendet wird, die auf ein Standardreferenzleck bezogen ist.

2. Ventilator nach Anspruch 1, wobei die mindestens eine erste Schnittstelle (13), um das unter Druck stehende Atemgas an einen Patienten abzugeben, in dem Ventilator (4) angeordnet ist.

3. Ventilator nach Anspruch 1 oder 2, wobei die Verarbeitungseinheit (11, 200) ferner einen Rechner (201) umfasst, der dafür ausgelegt ist, um eine Massenströmung für ein Standardleck unter Verwendung einer Formel, die aus der Bernoulli-Gleichung abgeleitet ist, zu berechnen, wobei die Formel wie folgt ist:

$$m = \int_{A_c} \rho u(r,x)\, dA_c$$

wobei $m$ die Massenströmung durch ein Rohr, p die Volumendichte des Fluids in dem Rohr, $u(r,x)$ das Geschwindigkeitsprofil für das Fluid in dem Rohr und $A_c$ die Querschnittsfläche für die Strömung ist, und wobei die berechnete Massenströmung durch den Druck für das unter Druck stehende Atemgas dividiert wird, um eine normalisierte Massenströmung zu erhalten.

4. Ventilator nach Anspruch 3, wobei der Rechner (201) dafür ausgelegt ist, um Werte für die Strömung eines Standardreferenzlecks aus einer Tabelle von Werten wiederaufzufinden, die die Strömungswerte eines Standardreferenzlecks bei einem bestimmten Druck des unter Druck stehende Atemgases darstellt.

5. Ventilator nach einem der Ansprüche 1 oder 4, wobei die Verarbeitungsmittel (11, 200) zusätzlich eine Speichereinheit (202) für eine spätere Analyse und Prüfung der gemessenen Signale umfassen, die einen Hinweis auf die augenblickliche Massenströmung des unter Druck stehenden Atemgases, auf den physiologischen Zustand des Patienten (1) und auf das Verhältnis zwischen dem gemessenen Signal, das einen Hinweis auf die augenblickliche Massenströmung für das unter Druck stehende Atemgas gibt, und einer Strömung eines Standardreferenzlecks geben.

6. Ventilator nach Anspruch 1, wobei die Verarbeitungseinheit (11, 200) zusätzlich eine erste Kommunikationsvorrichtung (206) umfasst, um mit einer äußeren Sensorvorrichtung (5, 6, 7, 8, 9, 10) zu kommunizieren.

7. Ventilator nach Anspruch 1, wobei die Verarbeitungseinheit (11, 200) ferner eine zweite Kommunikationsvorrichtung (207) für eine Kommunikation mit dem Ventilator (4) von einem externen Rechner aus umfasst, um Daten und Ergebnisse für eine Analyse und/oder Prüfung wiederaufzufinden.

8. Ventilator nach Anspruch 6 oder 7, wobei die erste oder zweite Kommunikationsvorrichtung (206, 207) eine leitungsgebundene oder leitungsfreie Kommunikationsvorrichtung (207) sein kann.

9. Ventilatonssystem, das umfasst:

- den mechanischen Ventilator (4) nach Anspruch 1,
- einen Schlauch (3), um das unter Druck stehende Atemgas, das mit dem mechanischen Ventilator (4) verbunden ist, zu leiten,
- eine Vorrichtung (2), die mit dem Schlauch (3) verbunden ist, um das unter Druck stehende Atemgas einem Patienten (1) zu verabreichen,
- mindestens eine Sensorvorrichtung (5, 6, 7, 8, 9, 10), die angeordnet ist, um mindestens ein Signal zu messen, das einen Hinweis auf die augenblickliche Strömung für das unter Druck stehende Atemgas gibt, und ferner angeordnet ist, um das Signal an den mechanischen Ventilator (4) zu senden.

10. Ventilator nach Anspruch 9, **dadurch gekennzeichnet, dass** die mindestens eine Sensorvorrichtung (5, 6, 7, 8, 9, 10) zum Messen eines Signals, das einen Hinweis auf die augenblickliche Strömung für das unter Druck stehende Atemgas gibt, in dem mechanischen Ventilator (4) oder in der Nähe von diesem oder in der Nähe der Vorrichtung (2) zum Verabreichen des unter Druck stehenden Atemgases an einen Patienten (1) angeordnet ist.

11. Ventilatonssystem nach einem der Ansprüche 9 bis 10, wobei die Verarbeitungseinheit (11, 200) ferner einen Rechner (201) umfasst, der dafür ausgelegt ist, um eine Massenströmung für ein Standardleck unter Verwendung einer Formel, die aus der Bernoulli-Gleichung abgeleitet ist, zu berechnen, wobei die Formel wie folgt ist:

$$m = \int_{A_c} \rho u(r,x) dAc$$

wobei $m$ die Massenströmung durch ein Rohr, p die Volumendichte des Fluids in dem Rohr, $u(r,x)$ das Geschwindigkeitsprofil für das Fluid in dem Rohr und $A_c$ die Querschnittsfläche für die Strömung ist, und wobei die berechnete Massenströmung durch den Druck für das unter Druck stehende Atemgas dividiert wird, um eine normalisierte Massenströmung zu erhalten.

12. Verfahren zum Bestimmen eines Lecks in einem Ventilator, wobei das Verfahren die folgenden Schritte umfasst:

- Messen der Massenströmung durch den Ventilator,
- Berechnen einer Strömung eines Standardreferenzlecks in dem Ventilator; **gekennzeichnet durch**:

Berechnen eines Verhältnisses zwischen der gemessenen Massenströmung **durch** den Ventilator und der berechneten Strömung eines Standardreferenzlecks in dem Ventilator; und
Bestimmen des Lecks aus dem Verhältnis.

13. Verfahren nach Anspruch 12, wobei auf der Grundlage des berechneten Verhältnisses zwischen der gemessenen Massenströmung durch den Ventilator und den Werten aus einer Standardleckberechnung für ein Standardleck in dem Ventilator eine Kompensation für die Differenz zwischen der gemessenen Massenströmung und der berechneten Standardleckströmung durchgeführt wird.

14. Verfahren nach Anspruch 12, wobei der Schritt des Messens der Massenströmung durch den Ventilator ferner die folgenden Unterschritte umfasst:

- Nehmen von Proben augenblicklicher Werte für die Massenströmung durch den Ventilator; und
- Berechnen eines Verhältnisses zwischen jedem genommenen Probenwert für die augenblickliche Massenströmung und einem entsprechenden Wert für die Standardleckströmung.

15. Verfahren nach Anspruch 12 oder 14, wobei die Unterschritte des Nehmens von Proben der augenblicklichen Werte für die Massenströmung durch den Ventilator und des Berechnens des Verhältnisses ferner die folgenden Schritte umfasst:

- Nehmen von Proben von Werten für die Massenströmung durch den Ventilator während einer vorgegebenen Zeitdauer;

- Berechnen eines Verhältnisses zwischen den abgetasteten Probenwerten der Massenströmung und entsprechenden Werten für eine Standardleckströmung während einer vorgegebenen Zeitdauer;

- Berechnen eines Mittelwerts für das Verhältnis, indem das Verhältnis über die vorgegebene Zeitdauer, die gemessen wird, integriert wird und durch die Anzahl abgetasteter Probenwerte dividiert wird; und

- Berechnen der Massenströmung durch den Ventilator unter Verwendung einer bekannten Beziehung zwischen dem Mittelwert für das Strömungsverhältnis und einer Standardleckströmung.

16. Verfahren nach Anspruch 12, wobei die Berechnung für die Standardleckströmung in dem Ventilator anhand der Bernoulli-Gleichung durchgeführt wird.

17. Verfahren nach Anspruch 12, wobei die Massenströmung für ein Standardleck unter Verwendung einer Formel, die aus der Bernoulli-Gleichung abgeleitet ist, berechnet wird, wobei die Formel wie folgt ist:

$$m = \int_{A_c} \rho u(r,x) dA_c$$

wobei $m$ die Massenströmung durch ein Rohr, p die Volumendichte des Fluids in dem Rohr, $u(r,x)$ das Geschwindigkeitsprofil für das Fluid in dem Rohr und $A_c$ die Querschnittsfläche für die Strömung ist, und wobei die berechnete Massenströmung durch den Druck für das unter Druck stehende Atemgas dividiert wird, um eine normalisierte Massenströmung zu erhalten.

18. Verfahren nach Anspruch 12, wobei der Schritt des Berechnens des Mittelwerts für das Verhältnis ferner die folgenden Unterschritte umfasst:

- Berechnen eines Volumens für die Einatmungs- und die Ausatmungsphasen eines Patienten;

- Bestimmen einer Volumendifferenz zwischen den Einatmungs- und den Ausatmungsphasen;

- Berechnen des tatsächlichen Durchflusses auf der Grundlage der Volumendifferenz;

- Berechnen eines Verhältnisses zwischen dem tatsächlichen Durchfluss auf der Grundlage der Volumendifferenz und einer Standardleckströmung; und

- Hinzufügen des Verhältnisses zwischen dem tatsächlichen Durchfluss auf der Grundlage der Volumendifferenz und einer Standardleckströmung und dem Mittelwert für das Verhältnis.

19. Computerprogramm, das dafür ausgelegt ist, das Verfahren nach Anspruch 12 auszuführen, und das die folgenden Sätze von Anweisungen umfasst zum:

- Erhalten von Daten, die einen Hinweis auf eine erste Massenströmung eines Atemgases durch das Ventilatorsystem geben;
- Erhalten einer zweiten Massenströmung für eine Standardleckströmung in dem Ventilatorsystem; **gekennzeichnet durch**:
- Berechnen eines Verhältnisses zwischen der ersten Massenströmung und der zweiten Standardleckströmung in dem Ventilatorsystem und;
- Bestimmen eines Lecks in dem Ventilatorsystem aus dem Verhältnis.

**Revendications**

1. Ventilateur (4) pour fournir un gaz respiratoire sous pression, comprenant :

- un générateur de flux (12) pour produire un gaz respiratoire sous pression à délivrer à un patient ;
- une première interface (13) raccordée audit générateur de flux (12) et aménagée pour recevoir ledit gaz respiratoire sous pression dudit générateur de flux (12) et délivrer ledit gaz respiratoire sous pression à un patient (1) ;
- au moins une seconde interface (15) raccordée à une unité de traitement (11, 200) et qui est à même de recevoir au moins un signal indicatif d'un flux mesuré de gaz respiratoire sous pression du patient (1) et délivrer le signal à ladite unité de traitement (11, 200) ; et

ladite unité de traitement (11, 200) pour commander la pression provenant du ventilateur (4) sur la base du signal indicatif du flux dudit gaz respiratoire sous pression reçu de ladite seconde interface (15), ladite unité de traitement (11, 200) étant aménagée pour compenser la fuite dans ledit ventilateur (4), **caractérisé en ce que**, pour compenser la fuite dans ledit ventilateur, on utilise un rapport entre ledit flux mesuré de gaz respiratoire sous pression et un flux apparenté à une fuite standard de référence.

2. Ventilateur selon la revendication 1, dans lequel ladite au moins une première interface (13) pour délivrer ledit gaz respiratoire sous pression à un patient est située dans ledit ventilateur (4).

3. Ventilateur selon les revendications 1 ou 2, dans lequel ladite unité de traitement (11, 200) comprend en outre un dispositif de calcul (201) qui est à même de calculer le flux massique pour une fuite standard en utilisant une formule dérivée de l'équation de Bernoulli, ladite formule étant la suivante:

$$m = \int_{A_c} \rho u(r,x) dAc$$

où m est le flux massique à travers un tuyau, p est la densité volumique du fluide dans le tuyau, u(r,x) est le profil de vitesse pour le fluide dans le tuyau et $A_c$ est la surface en coupe transversale pour le flux et dans lequel ledit flux massique calculé est divisé par la pression pour ledit gaz respiratoire sous pression afin d'obtenir un flux massique normalisé.

4. Ventilateur selon la revendication 3, dans lequel ledit dispositif de calcul (201) est à même de récupérer des valeurs pour ledit flux de fuite de référence standard dans une table de valeurs représentant lesdites valeurs de flux de fuite de référence standard à une certaine pression pour le gaz respiratoire sous pression.

5. Ventilateur selon l'une quelconque des revendications 1 ou 4, dans lequel ledit moyen de traitement (11, 200) comprend en outre une unité de stockage de données (202) pour une analyse et une inspection ultérieures des signaux mesurés indicatifs du flux massique instantané pour le gaz respiratoire sous pression, de l'état physiologique du patient (1) et dudit rapport entre le signal mesuré indicatif du flux massique instantané pour le gaz respiratoire sous pression et un flux de fuite de référence standard.

6. Ventilateur selon la revendication 1, dans lequel ladite unité de traitement (11, 200) comprend en outre un premier dispositif de communication (206) pour communiquer avec un dispositif de détection externe (5, 6, 7, 8, 9, 10).

7. Ventilateur selon la revendication 1, dans lequel ladite unité de traitement (11, 200) comprend en outre un second dispositif de communication (207) pour communiquer avec le ventilateur (4) à partir d'un dispositif de calcul externe permettant de récupérer des données et des résultats pour l'analyse et/ou l'inspection.

8. Ventilateur selon les revendications 6 ou 7, dans lequel ledit premier ou second dispositif de communication (206, 207) peut être un dispositif de communication câblé ou sans fil (207).

9. Système de ventilation comprenant :

- le ventilateur mécanique (4) selon la revendication 1,
- un tube (3) pour guider ledit gaz respiratoire sous pression raccordé audit ventilateur mécanique (4),
- un dispositif (2) raccordé audit tube (3) pour administrer ledit gaz respiratoire sous pression à un patient (1),
- au moins un dispositif de détection (5, 6, 7, 8, 9, 10) aménagé pour mesurer au moins un signal indicatif du flux instantané dudit gaz respiratoire sous pression et aménagé en outre pour envoyer ledit signal audit ventilateur mécanique (4).

10. Système de ventilation selon la revendication 9, **caractérisé en ce que** ledit au moins un dispositif de détection (5, 6, 7, 8, 9, 10) pour mesurer un signal indicatif du flux instantané pour ledit gaz respiratoire sous pression est situé dans ledit ventilateur mécanique (4) ou à proximité de celui-ci ou encore à proximité dudit dispositif (2) pour administrer ledit gaz respiratoire sous pression à un patient (1).

11. Système de ventilation selon l'une quelconque des revendications 9 à 10, dans lequel ladite unité de traitement (11, 200) comprend en outre un dispositif de calcul (201) qui est à même de calculer ledit flux massique pour une fuite standard en utilisant une formule dérivée de l'équation de Bernoulli, ladite formule étant la suivants :

$$m = \int_{A_c} \rho u(r,x) dAc$$

où m est le flux massique à travers un tuyau, p est la densité volumique du fluide dans le tuyau, u(r,x) est le profil de vitesse pour le fluide dans le tuyau et $A_c$ est la surface en coupe transversale pour le flux et dans lequel ledit flux massique calculé est divisé par la pression pour ledit gaz respiratoire sous pression afin d'obtenir un flux massique normalisé.

12. Procédé de détermination d'une fuite dans un ventilateur, comprenant les étapes consistant à :

- mesurer le flux massique à travers le ventilateur ;
- calculer un flux de fuite standard de référence dans ledit ventilateur,

**caractérisé en ce que** :

l'on calcule un rapport entre ledit flux massique mesuré à travers le ventilateur et ledit flux de fuite standard de référence calculé dans ledit ventilateur ; et
l'on détermine ladite fuite à partir dudit rapport.

**13.** Procédé selon la revendication 12, dans lequel, sur la base dudit rapport calculé entre le flux massique mesuré à travers le ventilateur et lesdites valeurs provenant d'un calcul de fuite standard pour une fuite standard dans ledit ventilateur, on effectue une compensation de la différence entre le flux massique mesuré et le flux de fuite standard calculé.

**14.** Procédé selon la revendication 12, dans lequel ladite étape de mesure du flux massique à travers le ventilateur comprend en outre les sous-étapes consistant à :

- échantillonner des valeurs instantanées pour le flux massique à travers le ventilateur ; et
- calculer un rapport entre ladite chaque valeur échantillonnée pour le flux massique instantané et une valeur correspondante pour le flux de fuite standard.

**15.** Procédé selon la revendication 12 ou 14, dans lequel lesdites sous-étapes d'échantillonnage desdites valeurs instantanées pour le flux massique à travers le ventilateur et le calcul dudit rapport comprennent en outre les étapes consistant à :

- échantillonner des valeurs pour le flux massique à travers le ventilateur au cours d'une période de temps prédéterminée ;
- calculer un rapport entre lesdites valeurs de flux massique échantillonnées et lesdites valeurs de flux de fuite standard correspondantes au cours de ladite période de temps prédéterminée ;
- calculer une valeur moyenne pour ledit rapport en intégrant le rapport sur la période de temps prédéterminée mesurée et en le divisant par le nombre de valeurs de flux échantillonnées ; et
- calculer le flux massique à travers le ventilateur en utilisant une relation connue entre ladite valeur moyenne pour le rapport de flux et un flux de fuite standard.

**16.** Procédé selon la revendication 12, dans lequel ledit calcul du flux de fuite standard dans ledit ventilateur est effectué à partir de l'équation de Bernoulli.

**17.** Procédé selon la revendication 12, dans lequel ledit flux massique pour une fuite standard est calculé en utilisant une formule dérivée de l'équation de Bernoulli, ladite formule étant la suivants :

$$m = \int_{A_c} \rho u(r,x) dAc$$

dans laquelle m est le flux massique à travers un tuyau, p est la densité volumique du fluide dans le tuyau, u(r,x) est le profil de vitesse pour le fluide dans le tuyau et $A_c$ est la surface en coupe transversale pour le flux et dans lequel ledit flux massique calculé est divisé par la pression pour ledit gaz respiratoire sous pression afin d'obtenir un flux massique normalisé.

**18.** Procédé selon la revendication 12, dans lequel ladite étape de calcul de la valeur moyenne pour ledit rapport comprend en outre des sous-étapes consistant à :

- calculer un volume pour les phases d'inspiration et d'expiration d'un patient ;
- déterminer une différence de volume entre lesdites phases d'inspiration et d'expiration ;
- calculer le débit réel sur la base de ladite différence de volume ;
- calculer un rapport entre ledit débit réel sur la base de ladite différence de volume et un flux de fuite standard ; et
- ajouter ledit rapport entre le débit réel basé sur ladite différence de volume et un flux de fuite standard et ladite valeur moyenne pour ledit rapport.

**19.** Programme informatique qui est à même d'effectuer le procédé de la revendication 12, comprenant des jeux d'instructions pour :

- obtenir des données indicatives d'un premier flux massique de gaz respiratoire à travers le système de ventilateur ;
- obtenir un second flux massique pour un flux de fuite standard dans ledit système de ventilateur ;

**caractérisé en ce que** :

- l'on calcule un rapport entre ledit premier flux massique et ledit second flux de fuite standard dans ledit système de ventilateur et
- l'on détermine une fuite dans ledit système de ventilateur à partir dudit rapport.

*Fig.1*

*Fig.2*

## Basic Flow

**310**

**320**

**330**

Normalized flow

0    10    20    30    40    50

**cmH2O**

Ipap 20 cmH₂O
Epap 4 cmH₂O

*Fig. 3*

**420**

VIVO

**402**

**430**

**410**

*Fig. 4*

500

510

*Fig. 5*

**Fig. 6**

**Fig. 7**

**EP 1 893 266 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6945248 B

- WO 0226460 A